(19) Europäisches Patentamt / European Patent Office / Office européen des brevets

(11) EP 2 427 051 B1

(12) **FASCICULE DE BREVET EUROPEEN**

(45) Date de publication et mention
de la délivrance du brevet:
**06.12.2017 Bulletin 2017/49**

(21) Numéro de dépôt: **10723787.7**

(22) Date de dépôt: **07.05.2010**

(51) Int Cl.:
*A01N 25/34* (2006.01)    *A01N 31/02* (2006.01)
*A01N 63/02* (2006.01)    *A01N 65/00* (2009.01)
*A01N 65/24* (2009.01)    *A01P 15/00* (2006.01)
*B42D 15/00* (2006.01)    *B42D 25/29* (2014.01)
*D21H 21/36* (2006.01)    *A61L 2/16* (2006.01)

(86) Numéro de dépôt international:
**PCT/IB2010/052028**

(87) Numéro de publication internationale:
**WO 2010/128487 (11.11.2010 Gazette 2010/45)**

(54) **SUPPORT D'INFORMATION PRESENTANT DES PROPRIETES ANTIVIRALES ET SON PROCEDE DE FABRICATION**

INFORMATIONSMEDIUM MIT ANTIVIRALEN EIGENSCHAFTEN UND VERFAHREN ZU SEINER HERSTELLUNG

INFORMATION MEDIUM HAVING ANTIVIRAL PROPERTIES, AND METHOD FOR MAKING SAME

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **07.05.2009 FR 0953053**

(43) Date de publication de la demande:
**14.03.2012 Bulletin 2012/11**

(73) Titulaire: **Oberthur Fiduciaire SAS**
**75008 Paris (FR)**

(72) Inventeur: **ROSSET, Henri**
**F-38730 Le Pin (FR)**

(74) Mandataire: **Nony**
**11 rue Saint-Georges**
**75009 Paris (FR)**

(56) Documents cités:
WO-A2-03/084326    WO-A2-2007/044398
US-A- 4 764 418    US-A- 5 968 538
US-A1- 2008 279 959

• CLARKE, N. M.; MAY, J. T.: "Effect of antimicrobial factors in human milk on rhinoviruses and milk-bourne cytomegalovirus in vitro", JOURNAL OF MEDICINAL MICROBIOLOGY, vol. 49, 30 juin 2000 (2000-06-30), pages 719-723, XP002558016, UK ISSN: 0022-2615
• LUCILLA SEGANTI ET AL: "Antiviral activity of lactoferrin towards naked viruses", BIOMETALS, KLUWER ACADEMIC PUBLISHERS, BO, vol. 17, no. 3, 1 juin 2004 (2004-06-01), pages 295-299, XP019232211, ISSN: 1572-8773
• LOIZZO, M. R.: "Phytochemical Analysis and in vitro Antiviral Activities of the Essential Oils of Seven Lebanon Species", CHEMISTRY & BIODIVERSITY, vol. 5, 20 mars 2008 (2008-03-20), pages 461-470, XP002558017, Zürich ISSN: 1612-1872, DOI: 10.1002/cbdv.200890045

Remarques:
Le dossier contient des informations techniques présentées postérieurement au dépôt de la demande et ne figurant pas dans le présent fascicule.

**Description**

L'invention concerne un support d'information destiné à être manipulé par un grand nombre d'utilisateurs, à l'image par exemple d'un billet de banque.

L'invention vise plus particulièrement à proposer un support d'information de ce type qui soit en outre avantageusement doté de propriétés antivirales, ainsi que son procédé de fabrication.

Dans les sociétés modernes, une quantité de plus en plus importante de supports destinés à transmettre des informations est manipulée quotidiennement et fréquemment par un grand nombre de personnes, pour lesquelles aucun contrôle sanitaire n'est exigé.

Or, ces individus, du fait de leur environnement, de leur activité professionnelle, de leur entourage et/ou de leur hygiène de vie, peuvent être porteurs de virus, susceptibles de générer des maladies épidémiques et pandémiques plus ou moins graves et, à ce titre, être susceptibles de contaminer tout support à leur contact.

Dans l'hypothèse où ce support est dédié de par sa nature à être à son tour diffusé, il devient alors lui-même un important véhicule de dissémination de virus et peut potentiellement provoquer des infections chez ceux qui le manipulent.

En outre, récemment, la possibilité d'une action terroriste par contamination virale de tels supports d'information n'étant plus à négliger, le risque lié à la manipulation de ces supports d'information devient particulièrement sensible.

En tant que monnaie d'échange lors des transactions commerciales, le billet de banque constitue l'un des supports d'information les plus manipulés au monde et représente, de ce fait, une menace potentielle pour la santé.

Il constitue en soi un vecteur potentiel de transmission des maladies et peut entraîner à l'égard de ceux qui le manipulent diverses infections susceptibles d'être aggravées en fonction des quantités d'agents pathogènes, de la virulence de l'échantillon, et de la résistance individuelle.

Par exemple, les billets de banque pourraient contribuer à favoriser les épidémies de grippe. Ainsi, une étude récente a mis en évidence que les virus de la grippe pouvaient survivre jusqu'à 17 jours sur un billet de banque conventionnel.

La demande de brevet WO 03/084326 envisage l'ajout d'un agent bactériostatique et/ou bactéricide et fongistatique et/ou fongicide dans un tel support d'information. La demande de brevet US 2008/0279959 décrit un support d'information avec une activité antimicrobienne. Il demeure toutefois un besoin de supports d'information efficaces pour lutter spécifiquement contre les virus, et éviter tout risque de transmission et de contamination virale.

Il demeure également un besoin de supports d'information présentant une activité antivirale tout en étant sans danger pour l'utilisateur, et notamment ne mettant pas en oeuvre, à titre de virucide, des composés toxiques et/ou dangereux.

Il est également souhaitable de disposer de supports d'information présentant une activité antivirale durable dans le temps.

Il est également souhaitable de disposer de supports d'information dont l'activité antivirale reste préférentiellement attachée au support d'information.

L'invention vise à fournir un support d'information présentant des propriétés antivirales et satisfaisant à ces besoins.

La Demanderesse, après avoir testé de nombreuses compositions virucides, est parvenue, de façon surprenante, à résoudre les problèmes posés en traitant le support d'information à l'aide d'un virucide d'origine naturelle.

Ainsi, l'invention concerne, selon un de ses aspects, un support d'information selon la revendication 1.

Un tel support peut ainsi être qualifié de support d'information à propriétés antivirales, c'est-à-dire doté d'une aptitude à être actif contre les virus.

Le virucide utilisé selon l'invention ne présente avantageusement pas de toxicité particulière au regard des individus amenés à les manipuler et n'est pas soumis à des directives particulières.

L'invention concerne également, selon un autre de ses aspects, un procédé de fabrication d'un support d'information tel que défini précédemment, comprenant au moins l'étape consistant à mettre en contact, en présence d'au moins un agent humectant, un support de base avec ledit virucide d'origine naturelle, ou notamment un précurseur de celui-ci, dans des conditions propices à l'incorporation de celui-ci au niveau dudit support.

L'invention concerne également, selon encore un autre de ses aspects, un procédé de fabrication d'un support d'information antiviral, caractérisé en ce qu'au moins un virucide d'origine naturelle est synthétisé *in situ* au niveau d'un support de base formé par exemple de matériaux cellulosiques et/ou plastiques.

Comme il ressort de ce qui suit, les propriétés virucides dudit support lui sont conférées via le traitement de celui-ci avec une composition comprenant au moins ledit virucide.

Ce traitement peut être réalisé au cours du procédé de préparation dudit support mais également consécutivement.

Ainsi, les propriétés virucides dudit support selon l'invention peuvent lui être conférées lors de son impression avec une encre comprenant au moins ledit virucide d'origine naturelle, ou encore par dépôt d'un vernis, notamment de surimpression, comprenant au moins un tel virucide d'origine naturelle.

En conséquence, la présente invention vise également un support d'information conforme à l'invention dont les propriétés virucides sont conférées *via* une encre, imprimée sur ledit support, ladite encre comprenant au moins ledit virucide d'origine naturelle.

Elle vise en outre un support d'information conforme à l'invention, dont les propriétés virucides sont conférées *via* la présence d'un vernis, notamment de surimpression, déposé sur ledit support, ledit vernis comprenant au moins ledit virucide d'origine naturelle.

## Support d'information contenant au moins un virucide d'origine naturelle

### Support d'information

Comme indiqué précédemment, les supports d'information plus particulièrement considérés dans le cadre de la présente invention sont des supports d'information destinés à être manipulés relativement fréquemment.

Au sens de l'invention, un « support d'information destiné à être manipulé relativement fréquemment » est un support manipulé au moins deux fois manuellement par un même individu ou au moins deux individus distincts. Une manipulation manuelle peut se composer d'au moins un contact, par exemple une saisie, par au moins une partie d'une main.

Ainsi, les supports à usage unique ne sont par exemple pas considérés comme des supports d'information conformes à l'invention.

Les supports d'information conformes à l'invention sont plus particulièrement des supports destinés à un usage en atmosphère ambiante.

En d'autres termes, les supports considérés selon l'invention ne sont, d'une manière générale, pas dédiés à une utilisation en milieu liquide et plus particulièrement aqueux.

Le support d'information destiné à être manipulé relativement fréquemment conforme à l'invention peut être notamment un document de sécurité comportant au moins un élément de sécurité.

Le document de sécurité, ainsi que les éléments de sécurité qu'il comporte comme par exemple un fil de sécurité, un filigrane, une impression, un patch et/ou un foil, peuvent comporter un ou plusieurs éléments de sécurité tels que définis ci-après.

Parmi les éléments de sécurité, certains sont détectables à l'oeil, en lumière du jour ou en lumière artificielle, sans utilisation d'un appareil particulier. Ces éléments de sécurité comportent par exemple des fibres ou planchettes colorées, des fils imprimés ou métallisés totalement ou partiellement. Ces éléments de sécurité sont dits de premier niveau.

D'autres types d'éléments de sécurité sont détectables seulement à l'aide d'un appareil relativement simple, tel qu'une lampe émettant dans l'ultra-violet (UV) ou l'infra-rouge (IR). Ces éléments de sécurité comportent par exemple des fibres, des planchettes, des bandes, des fils ou des particules. Ces éléments de sécurité peuvent être visibles à l'oeil nu ou non, étant par exemple luminescents sous un éclairage d'une lampe de Wood émettant dans une longueur d'onde de 365 nm. Ces éléments de sécurité sont dits de deuxième niveau.

D'autres types d'éléments de sécurité encore nécessitent pour leur détection un appareil de détection plus sophistiqué. Ces éléments de sécurité sont par exemple capables de générer un signal spécifique lorsqu'ils sont soumis, de manière simultanée ou non, à une ou plusieurs sources d'excitation extérieure. La détection automatique du signal permet d'authentifier, le cas échéant, le document. Ces éléments de sécurité comportent par exemple des traceurs se présentant sous la forme de matières actives, de particules ou de fibres, capables de générer un signal spécifique lorsque ces traceurs sont soumis à une excitation optronique, électrique, magnétique ou électromagnétique. Ces éléments de sécurité sont dits de troisième niveau.

Les éléments de sécurité présents au sein du document de sécurité et des éléments qu'il comporte, peuvent présenter des caractéristiques de sécurité de premier, de deuxième ou de troisième niveau.

Le support d'information conforme à l'invention peut comporter un substrat comportant des fibres papetières connues de l'homme du métier, par exemple des fibres cellulosiques (en particulier des fibres de coton) et/ou des fibres organiques naturelles autres que cellulosiques et/ou des fibres synthétiques, par exemple telles que des fibres de polyester ou de polyamide, et/ou éventuellement des fibres minérales, par exemple telles que des fibres de verre.

Selon un mode de réalisation, le support d'information conforme à l'invention est à base de matériaux, notamment de fibres, cellulosiques, et en particulier de papier.

Selon un autre mode de réalisation, le support d'information conforme à l'invention est à base de fibres organiques naturelles autres que cellulosiques.

Selon encore un autre mode de réalisation, le support d'information conforme à l'invention est à base de matériaux plastiques, et notamment de fibres synthétiques ou d'une feuille plastique.

Le support peut être également un film plastique, et notamment un film bi-étiré à base de polyéthylène à l'image du matériau Polyart® commercialisé par la société Arjobex. Il s'agit plus particulièrement d'une feuille comportant un support coextrudé, réalisé à partir d'au moins un matériau polymère, comportant par exemple une couche de coeur et au moins une couche de peau, la couche de coeur comportant des vides.

Le support peut être également un support multicouche, notamment laminé ou contrecollé. Ledit support multicouche comprend en particulier au moins une couche à base de matériaux cellulosiques ou plastiques tels que décrits précédemment.

Selon encore un autre mode de réalisation, le support d'information conforme à l'invention est à base de fibres minérales.

Le support d'information considéré selon l'invention est un passeport, une carte d'identité, un permis de conduire, une carte d'accès, une carte de fidélité, une carte de photocopie, une carte de cantine, une carte à jouer, une carte à collectionner, un moyen de paiement, notamment une carte de paiement, un billet de banque, un bon d'achat ou un reçu, un ticket d'accès à des manifestations culturelles ou sportives, un certificat d'authenticité, ou encore un emballage, un livre, une carte géographique, une étiquette, une enveloppe ou un magasine.

De préférence, le support d'information conforme à l'invention est un document de sécurité, et en particulier un billet de banque.

Virucide d'origine naturelle

Le support d'information conforme à l'invention contient au moins un virucide d'origine naturelle, ledit virucide étant la monolaurine.

Au sens de la présente invention, le terme « virucide » désigne tout composé possédant la capacité de tuer ou d'inhiber les virus.

Le virucide selon la présente invention est plus particulièrement dédié à tuer et/ou à inhiber un virus pathogène à l'égard des mammifères et plus particulièrement de l'Homme. De tels virus peuvent être des virus nus ou des virus enveloppés.

A titre représentatif des virus pathogènes pour l'Homme susceptibles d'être considérés selon l'invention, on peut plus particulièrement citer les rétrovirus, les cytomégalovirus, les rotavirus, les paramyxovirus, les poliovirus, les hantavirus, les virus coxsackie, le virus de l'encéphalomyocardite, les picornavirus dont les rhinovirus, les virus à ADN ou à ARN notamment les flaviviridae, le virus du SIDA, les virus de la grippe, le virus de la variole, le virus de la fièvre jaune, le virus de l'hépatite C, les virus de l'herpès, le virus d'Epstein-Barr, le virus varicelle-zona, le virus de la rubéole, ou encore le virus simien 40 ou SV40.

Par « virucide d'origine naturelle », on entend désigner tout virucide pré-existant dans la nature ou pouvant être synthétisé à partir de composés naturels existant dans la nature.

Les virucides d'origine naturelle peuvent ainsi être obtenus soit par extraction et purification à partir d'un milieu naturel les contenant, soit par synthèse à partir de composés naturels.

A titre d'exemple de tels virucides, on peut notamment citer la monolaurine qui peut être obtenue par synthèse à partir de glycérol et d'acide laurique.

Dans le cas de cette seconde alternative, le glycérol et l'acide laurique constituent au sens de l'invention un précurseur de virucide dans la mesure où ils permettent, à l'issue du procédé selon l'invention de générer un support à propriétés antivirales.

Plus précisément, le terme « précurseur » désigne, selon l'invention, un composé qui est à même, lors des étapes du procédé selon l'invention, soit par transformation soit par réaction avec un autre composé qui lui est associé, et donc également qualifié de précurseur, de générer le virucide attendu.

On peut décrire comme virucides d'origine naturelle la monolaurine, la lactoferrine et les huiles essentielles présentant une activité antivirale, comme par exemple l'huile essentielle de laurier.

Au sens de l'invention, le terme monolaurine entend désigner à la fois la monolaurine pré-existant naturellement et celle obtenue par synthèse à partir de glycérol et d'acide laurique.

Ce type de virucide d'origine naturelle a en effet été identifié comme présentant des propriétés particulièrement avantageuses pour la préparation de supports d'informations tels que considérés dans le cadre de la présente invention.

Le support d'information conforme à l'invention contient une quantité efficace d'au moins ledit virucide d'origine naturelle, c'est-à-dire une quantité suffisante de celui-ci pour doter le support d'information l'incorporant de propriétés antivirales.

Selon un mode de réalisation, il peut notamment s'agir d'une quantité suffisante dudit virucide d'origine naturelle pour conférer audit support d'information l'incorporant une activité antivirale supérieure à 1 log, suivant le protocole de mesure décrit dans les exemples.

Pour des raisons évidentes, la quantité de virucide d'origine naturelle à mettre en oeuvre selon l'invention dépend notamment de la nature dudit virucide et/ou de la nature du support d'information et peut donc varier dans une large mesure.

L'homme du métier peut aisément, sur la base de ses connaissances générales, déterminer les quantités appropriées. L'ajustement de la quantité de virucide d'origine naturelle fait partie des compétences de l'homme du métier.

Le support d'information conforme à l'invention contient de 0,1 à 2 % en poids sec, par exemple de 0,5 à 1,5 % en poids sec, dudit virucide d'origine naturelle par rapport à son poids total.

Selon un mode de réalisation, le support d'information conforme à l'invention peut contenir en outre d'autres composés actifs additionnels, présentant ou non une activité antivirale.

Il peut notamment contenir en outre des biocides, et par exemple des biocides de type bactériostatique et/ou bactéricide et/ou fongistatique et/ou fongicide.

Selon un autre mode de réalisation, le virucide d'origine naturelle requis selon l'invention peut présenter lui-même, outre son activité antivirale, au moins une autre activité biologique.

Ainsi, le virucide d'origine naturelle requis selon l'invention peut par exemple présenter en outre une activité bactériostatique, bactéricide, fongistatique ou fongicide, et plus particulièrement une activité bactériostatique ou bactéricide.

Agent Humectant

Au sens de l'invention, un agent humectant est un composé apte à procurer un effet d'hydratation ou encore hygroscopique.

Contre toute attente, les inventeurs ont constaté que la présence d'un tel composé permet de stimuler l'activité antivirale du virucide d'origine naturelle associé, et donc d'accroître l'activité antivirale manifestée par un support d'information conforme à l'invention incorporant ces 2 composés.

Des agents humectants peuvent être les composés de type polyol, tels que, par exemple, la glycérine, dite encore glycérol, le propylène glycol, le polyéthylène glycol, le butylène glycol, le triacétate de glycéryle, ou encore le sorbitol.

Selon l'invention, l'agent humectant considéré est le glycérol.

D'autres agents humectants décrits sont les composés suivants :

- acide pidolique (PCA) et ses dérivés (arginine PCA, copper PCA, ethylhexyl PCA, lauryl PCA, magnesium PCA, sodium PCA, zinc PCA...),
- le gluconate de calcium,
- le fructose, le glucose, l'isomalt, le lactose, le maltitol, le mannitol, le polydextrose, le sorbitol, le saccharose ou le xylitol,
- l'acide glycyrrhizique et ses dérivés,
- l'histidine,
- l'acide hyaluronique et ses sels tels que l'hyaluronate de sodium,
- les hydrolysats de soie, de kératine ou de soja
- le phytantriol,
- la soie, ou
- l'urée.

Le support d'information conforme à l'invention peut contenir de 0,5 à 4 % en poids sec, par exemple de 1 à 3 % en poids sec d'agent(s) humectant(s), et notamment de glycérol, par rapport à son poids total.

Selon un mode de réalisation préféré, l'agent humectant est présent dans le support d'information conforme à l'invention en un rapport pondéral masse d'agent(s) humectant(s) sur masse de virucide(s) au moins égal à 1.

Selon un mode de réalisation particulier, le support d'information conforme à l'invention peut contenir au moins un virucide selon la présente invention, au moins un agent humectant, étant selon la présente invention du glycérol, et en outre au moins un biocide bactériostatique et/ou bactéricide ou un biocide fongistatique et/ou fongicide.

Selon un autre mode de réalisation particulier, le support d'information conforme à l'invention peut contenir au moins un virucide selon la présente invention, au moins un agent humectant, étant selon la présente invention du glycérol, et en outre au moins un biocide bactériostatique et/ou bactéricide et au moins un biocide fongistatique et/ou fongicide.

**Procédé de fabrication**

Un autre objet de l'invention concerne un procédé de fabrication d'un support d'information tel que défini précédemment.

Selon un premier mode de réalisation, il peut s'agir d'un procédé de fabrication comprenant au moins l'étape consistant à mettre en contact, en présence d'au moins ledit agent humectant, un support de base avec un tel virucide d'origine naturelle dans des conditions propices à l'incorporation de celui-ci au niveau dudit support.

Afin de se placer dans des conditions encore plus propices à l'incorporation dudit virucide d'origine naturelle au niveau dudit support, on peut être amené à utiliser des émulsions ou des solutions particulières, par exemple telles que des solutions ammoniacales ou de préférence à base de 2-amino-2-méthyl-1-propanol qui présente l'avantage de ne pas engendrer de dégagement odorant.

Selon une variante de réalisation, l'agent humectant peut être présent dans une telle émulsion.

Le virucide d'origine naturelle est tel que défini précédemment.

L'agent humectant est tel que défini précédemment.

La mise en contact, et l'incorporation, dudit virucide dans le support de base peut se faire de diverses manières :

- par immersion dudit support de base dans une solution dudit virucide,
- par pulvérisation dudit support de base avec une solution dudit virucide,
- par impression dudit support de base à l'aide d'une encre contenant ledit virucide,
- par surfaçage dudit support de base avec une préparation contenant ledit virucide et un agent de surfaçage aqueux, l'agent de surfaçage aqueux incorporant de préférence de la glycérine comme plastifiant,
- par couchage dudit support de base avec une solution de couchage contenant ledit virucide,
- par dépôt sur ledit support de base, d'un vernis de surimpression contenant ledit virucide, et
- par enduction de microcapsules ou de cyclodextrine contenant ledit virucide sur ledit support de base.

L'agent humectant est avantageusement présent dans la composition ou la solution comprenant ledit virucide.

En particulier, lesdites mises en contact et incorporation de monolaurine peuvent être favorisée par l'utilisation d'une émulsion de monolaurine.

Selon un autre de ses aspects, l'invention concerne un procédé de fabrication d'un support d'information dans lequel au moins un virucide d'origine naturelle est synthétisé *in situ* au niveau d'un support de base formé par exemple de matériaux cellulosiques et/ou plastiques.

Selon une variante de réalisation, ce procédé peut comprendre en outre la mise en oeuvre d'un agent humectant, notamment tel que défini ci-dessus.

Cette synthèse peut notamment être réalisée lors d'au moins une étape du type de celles proposées ci-dessus pour la mise en contact du virucide avec le support de base. Dans ce cas de figure, la mise en contact est établie entre le support et le ou les précurseur(s) du virucide.

Le virucide d'origine naturelle est tel que défini précédemment.

Cette variante de réalisation est particulièrement adaptée lorsque le virucide d'origine naturelle est par exemple aisément accessible par synthèse, de préférence à des coûts en outre avantageux.

Ainsi, il pourra s'agir par exemple de la monolaurine synthétisée *in situ* par réaction d'acide laurique et de glycérol en présence d'un catalyseur.

La monolaurine est en effet disponible par ailleurs commercialement mais à des prix relativement élevés. Sa synthèse *in situ* selon cette variante de réalisation permet donc de la mettre en oeuvre dans un support d'information à un coût réduit.

En particulier lesdites mises en contact et incorporation d'acide laurique peuvent être favorisées par l'utilisation d'une solution d'acide laurique, notamment telle qu'une solution ammoniacale ou de préférence à base de 2-amino-2-méthyl-1-propanol qui présente l'avantage de ne pas engendrer de dégagement odorant.

Selon ce deuxième mode de réalisation, le procédé de fabrication peut comprendre au moins :

a) le dépôt en surface dudit support de base, en présence d'un catalyseur, d'une composition contenant au moins de l'acide laurique et du glycérol, et

b) le traitement consécutif de ce support à une température propice à la synthèse de monolaurine.

Selon une variante de réalisation, le catalyseur est présent dans le support de base mis en oeuvre en étape a).

Cette variante est notamment adaptée lorsque le support d'information se présente sous la forme d'une feuille de papier.

En effet, le catalyseur peut être introduit en masse dans la suspension fibreuse de base pendant l'étape de formation de la feuille tandis que l'acide laurique et le glycérol peuvent être présents dans une solution de traitement et être introduits ainsi en surface du support.

Selon une autre variante de réalisation, ce catalyseur peut être présent dans la composition contenant l'acide laurique et le glycérol.

Par ailleurs, selon ce deuxième mode de réalisation, ledit support de base peut être un support à base de matériaux cellulosiques, notamment du papier, et les étapes a) et b) peuvent être réalisées simultanément aux étapes requises pour le couchage, l'enduction ou le surfaçage dudit support de base.

En particulier, l'étape b) peut être réalisée simultanément à l'étape de séchage du papier couché, enduit ou surfacé.

Cette étape de séchage peut notamment être réalisée à une température supérieure ou égale à 80 °C, par exemple supérieure ou égale à 90 °C, de préférence supérieure ou égale à 100 °C.

Ce mode de réalisation est particulièrement avantageux dans la mesure où il permet d'incorporer le virucide *via* un procédé conventionnel de fabrication d'un support d'information, notamment de type papier, c'est-à-dire concomitamment aux étapes conventionnelles de fabrication.

Il ne nécessite donc avantageusement pas d'étape additionnelle autre que celles requises pour la fabrication du support.

Selon une variante de réalisation, ce procédé peut être réalisé en présence d'un agent anti-mousse.

Plus particulièrement, il s'agit d'un composé commercialisé sous le nom Aerotech 3514 (KEMIRA CHIMIE SA) et qui est formé d'un mélange d'huiles minérales et de tensioactifs non ioniques.

Un tel composé peut être introduit à un concentration comprise entre 0,01% et 0,30%, de préférence entre 0,04% et 0,20%, et plus préférentiellement entre 0,04% et 0,12% par rapport au poids total du mélange d'acide laurique et de glycérol.

Comme indiqué précédemment, la synthèse de monolaurine à partir d'acide laurique et de glycérol se déroule en présence d'un catalyseur.

A titre d'exemple de catalyseur convenant plus particulièrement à la catalyse de cette réaction, on peut notamment citer les zéolites, et par exemple la zéolite A commercialisée par la société FMC Foret ou les lipases.

Dans le cas où le catalyseur est une lipase, on peut notamment se référer aux conditions de réaction décrites par Pereira C.C.B., Da Silva M.A.P. et Langone M.A.P. dans la publication « Enzymatic synthesis of monolaurin » (Applied biochemistry and Biotechnology, 2004, vol. 113-116, P.433-445).

A titre de lipase convenant plus particulièrement dans le cadre de la présente invention, on peut par exemple citer les lipases commercialisées sous les références Liposyme RM IM®, Lipozyme TL IM® et Resinase A2C® par la société NOVOZYMES.

Le support d'information conforme à l'invention peut contenir de 0,5 à 3 % en poids sec, par exemple de 0,5 à 2 % en poids sec, de catalyseur par rapport à son poids total.

Le catalyseur, par exemple la zéolite, peut être introduit à raison d'au moins 2 % en poids, par exemple au moins 5 % en poids, par rapport au poids total du mélange d'acide laurique et de glycérol.

Selon une première variante de réalisation, l'acide laurique et le glycérol peuvent être introduits en mélange équimolaire.

Selon une deuxième variante de réalisation, le glycérol peut être introduit en excès par rapport à l'acide laurique.

Selon cette deuxième variante, du glycérol en excès résiduel reste donc présent dans le support à la fin de la réaction.

Comme mentionné précédemment, ce glycérol résiduel peut jouer le rôle d'agent humectant et augmenter les propriétés antivirales.

Un exemple de procédé de préparation *in situ* de monolaurine figure dans l'exemple 3 ci-après.

Les exemples, non limitatifs, suivants permettront de mieux comprendre comment l'invention peut être mise en pratique et ses avantages.

## Exemples

### Exemple 1 comparatif :

On forme une feuille de papier sur une machine à papier dite de forme ronde avec une toile comportant un motif permettant de faire un filigrane, ce papier pouvant convenir comme papier pour fabriquer un billet de banque, de la façon suivante :

- on met en suspension dans de l'eau une pâte de fibres de coton, on raffine cette suspension à 60° SCHOEPPER-RIEGLER,
- on ajoute un agent de résistance humide, environ 2,5 % en poids sec d'une résine poly(amide-amine-epychlorhydrine), exprimés par rapport aux fibres de coton,
- on introduit également dans cette suspension des planchettes iridescentes,
- on introduit lors de la formation de la feuille, un fil de sécurité microimprimé dit « window thread », selon les techniques antérieures connues de manière à faire apparaître ce fil dans certaines fenêtres à la surface du papier. Une méthode utilisable pour introduire ce fil est décrite par exemple dans le brevet EP 0 059 056, et
- on sèche la feuille vers 100 °C.

**Exemple 2** (hors invention)

On reprend un support de l'exemple 1 que l'on enduit avec une préparation réalisée en milieu aqueux qui comporte :

- 31,2 parts en poids sec de glycérine,
- 18,8 parts en poids sec de lactoferrine,
- 31,2 parts en poids sec d'un liant PVA et
- 18,8 parts en poids sec de zéolite (zéolite A).

La concentration de lactoferrine par rapport à la solution de couchage totale est réglée à 4,7 % en poids.
Une fois enduit, le papier comporte un taux en poids sec de lactoferrine d'environ 0,98 g/m$^2$.

**Exemple 3**

On reprend un support de l'exemple 1 que l'on enduit avec une préparation réalisée en milieu aqueux qui comporte :

- 31, 2 parts en poids sec de glycérine,
- 18,8 parts en poids sec d'acide laurique,
- 31,2 parts en poids sec d'un liant PVA, et
- 18,8 parts en poids sec de zéolite (zéolite A).

Les concentrations de glycérine et d'acide laurique par rapport à la solution de couchage totale sont réglées respectivement à 6,24 et 3,76 % en poids.
Une fois enduit, le papier comporte un taux en poids sec de monolaurine d'environ 1,03 g/m$^2$.

**Exemple 4**

On reprend un support de l'exemple 1 que l'on enduit avec une préparation réalisée en milieux aqueux qui comporte :

- 31,2 parts en poids sec de glycérine,
- 18,8 parts en poids sec de monolaurine,
- 31, 2 parts en poids sec d'un liant PVA, et
- 18, 8 parts en poids sec de zéolite (zéolite A).

La concentration de monolaurine par rapport à la solution de couchage totale est réglée à 3,76 % en poids.
Une fois enduit, le papier comporte un taux en poids sec de monolaurine d'environ 1,13 g/m$^2$.

**Exemple 5** (hors invention)

On reprend un support de l'exemple 1 que l'on enduit avec une préparation réalisée dans une dispersion de polyuréthanes qui comporte :

- 56,4 parts en poids sec de polyuréthane,
- 5,6 parts en poids sec de silice colloïdale,
- 33,8 parts en poids sec de glycérine,
- 3,8 parts en poids sec d'huile essentielle de laurier noble, et
- 0,4 parts en poids sec d'émulsifiant (éthoxylate d'alcool gras).

La concentration d'huile essentielle de laurier par rapport à la solution de couchage totale est réglée à 1,6 % en poids.
Le pH de la solution de couchage est fixé à 8,4.
Une fois enduit, le papier comporte un taux en poids sec d'huile essentielle de laurier noble d'environ 0,19 g/m$^2$.

## Exemple 6

On reprend un support de l'exemple 1 que l'on imprègne avec une préparation réalisée en milieu aqueux qui comporte :

- 40 Kg de liant PVA,

Les PVA sont cuits et de l'eau est ajoutée pour un volume final de 950 L.

- 25 Kg de glycérine,
- 20 Kg d'acide laurique,
- 2 Kg de zéolite (zéolite A), et
- 10 L d'AMP90.

## Tests et résultats

### 1. Activité antiphagique

Le test de l'activité antiphagique, propre à la Demanderesse, est basé sur la norme JIS L 1902 modifiée, ou encore sur la norme ISO 20743 modifiée, sur les phages MS2, qui sont réputés être très résistants, et appliquée sur des temps d'action compris entre 18 et 24 heures.

Le principe est le suivant : des phages MS2 sont déposés sur les supports à tester, puis le nombre de phages MS2 actifs est évalué une première fois à t=0h, et une deuxième fois à t=24h.

Pour évaluer le nombre de phages MS2 actifs sur les supports à tester à un temps donné, on met ces supports en présence de bactéries particulières qui présentent la propriété d'être des hôtes des phages MS2 : la mesure du nombre de plages de lyses (ou pfp) après culture permet alors de remonter à la quantité de phages MS2 recherchée.

On peut ainsi en déduire une activité antiphagique (notée A), définie comme suit :

$$A = [\text{moy log } (C_{24}) - \text{moy log } (C_0)] - [\text{moy log } (E_{24}) - \text{moy log } (E_0)],$$

formule dans laquelle $E_{24}$ correspond au nombre de plages de lyses à 24h et $E_0$ correspond au nombre de plages de lyses juste après sa mise en contact avec le support testé.

Les conditions expérimentales sont les suivantes :

- Le diluant utilisé est le peptone/sel (de référence DIFCO, 1897-17) et la souche bactérienne utilisée est *Escherichia coli* K12, qui est une souche hôte des phages MS2.
- Le support témoin est un textile 100 % coton non traité.
- On dépose 200 μL d'une suspension de phages à 1 x $10^5$ pfp/mL.

Les résultats sont reportés ci-après.

**Exemples 2 à 4** :

| Temps d'incubation | | 0h | | | | 24h | | | |
|---|---|---|---|---|---|---|---|---|---|
| Ref Echantillon | éprouvette | $C_0$ (pfp/g) | log $(C_0)$ | Ecart type | moy log $(C_0)$ | $C_{24}$ (pfp/g) | log $(C_{24})$ | Ecart type | moy log $(C_{24})$ |
| **Témoin** | 1 | 256 000 | 5,41 | 0,07 | **5,36** | 38 600 | 4,59 | 0,00 | **4,59** |
| | 2 | 207 000 | 5,32 | | | 39 100 | 4,59 | | |

| Temps d'incubation | | 0 h | | | | 24 h | | | |
|---|---|---|---|---|---|---|---|---|---|
| Réf. Echantillon | éprouvette | $E_0$ (pfp/g) | log $(E_0)$ | Ecart type | moy log $(E_0)$ | $E_{24}$ (pfp/g) | log $(E_{24})$ | Ecart type | moy log $(E_{24})$ |
| **Exemple 2** | 1 | 84 000 | 4,92 | 0,01 | **4,92** | 800 | 2,90 | 0,21 | **2,75** |
| | 2 | 82 000 | 4,91 | | | 400 | 2,60 | | |
| **Exemple 3** | 1 | 223 000 | 5,35 | 0,15 | 5,24 | 370 | 3,14 | 0,08 | 3,19 |
| | 2 | 136 000 | 5,13 | | | 1 760 | 3,25 | | |
| **Exemple 4** | 1 | 74 000 | 4,87 | 0,11 | **4,95** | 1 100 | 3,04 | 0,08 | **2,98** |
| | 2 | 106 000 | 5,03 | | | 840 | 2,92 | | |

On en déduit les activités antiphagiques suivantes :

$$A_{\text{exemple 2}} = -0,77 - (-2,17) = 1,40 \text{ log}$$

$$A_{\text{exemple 3}} = -0,77 - (-2,05) = 1,28 \text{ log}$$

$$A_{\text{exemple 4}} = -0,77 - (-1,97) = 1,20 \text{ log}$$

## Exemple 5 :

| Temps d'incubation | | 0h | | | | 24h | | | |
|---|---|---|---|---|---|---|---|---|---|
| Réf Echantillon | éprouvette | $C_0$ (pfp/g) | log $(C_0)$ | Ecart type | moy log $(C_0)$ | $C_{24}$ (pfp/g) | log $(C_{24})$ | Ecart type | moy log $(C_{24})$ |
| **Témoin** | 1 | 2 728 000 | 6,44 | 0,05 | **6,47** | 830 000 | 5,92 | 0,15 | **6,02** |
| | 2 | 3 160 000 | 6,50 | | | 1 350 000 | 6,13 | | |

| Temps d'incubation | | 0 h | | | | 24 h | | | |
|---|---|---|---|---|---|---|---|---|---|
| Réf. Echantillon | éprouvette | $E_0$ (pfp/g) | log $(E_0)$ | Ecart type | moy log $(E_0)$ | $E_{24}$ (pfp/g) | log $(E_{24})$ | Ecart type | moy log $(E_{24})$ |
| **Exemple 5** | 1 | 1 735 000 | 6,24 | 0.01 | **6,24** | 35 000 | 4,54 | 0,19 | **4,68** |
| | 2 | 1 772 000 | 6,25 | | | 64 000 | 4,81 | | |

On en déduit de même l'activité antiphagique suivante :

$$A_{exemple\ 5} = -\ 0,45 - (-1,56) = 1,11\ log.$$

Ces essais démontrent par conséquent que les supports obtenus conformément à l'invention présentent effectivement une activité antivirale significative.

**Exemple 6 :**

| Temps d'incubation | 0h | | | | | 24h | | | |
|---|---|---|---|---|---|---|---|---|---|
| Réf Echantillon | éprouvette | $C_0$ (pfp/g) | log $(C_0)$ | Ecart type | moy log $(C_0)$ | $C_{24}$ (pfp/g) | log $(C_{24})$ | Ecart type | moy log $(C_{24})$ |
| **Témoin** | 1 | 17 000 | 4,23 | 0,12 | **4,31** | 5 400 | 3.73 | 0,19 | **3,60.** |
| | 2 | 25 000 | 4,40 | | | 2 900 | 3,46 | | |

| Temps d'incubation | 0 h | | | | | 24 h | | | |
|---|---|---|---|---|---|---|---|---|---|
| Réf. Echantillon | éprouvette | $E_0$ (pfp/g) | log $(E_0)$ | Ecart type | moy log $(E_0)$ | $E_{24}$ (pfp/g) | log $(E_{24})$ | Ecart type | moy log $(E_{24})$ |
| **Exemple 6** | 1 | 23 000 | 4,36 | 0,00 | **4,36** | 0 | - | - | **Résultats** |
| | 2 | 23 000 | 4,36 | | | 60 | 1,78 | | **hétérogène** |

Des valeurs présentées ci-dessus, on peut noter que l'éprouvette 1 est totalement phagicide, et l'on peut calculer l'activité antiphagique de l'éprouvette 2 comme suit :

$$A_{exemple\ 6} = -\ 0,71 - (-2,58) = 1,87\ log.$$

Ces essais démontrent par conséquent que les supports obtenus conformément à l'invention présente effectivement une activité antivirale significative.

2. Activité bactéricide/bactériostatique

Des tests antibactériens ont également été réalisés sur le support d'information obtenu selon l'exemple 6, à l'aide de 2 souches bactériennes, à savoir *Staphylococcus aureus* CIP 4.83 et *Klebsiella pneumoniae* 368 CIP.
Le test de l'activité bactéricide/bactériostatique, propre à la Demanderesse, est basé sur la norme sur la norme ISO 20743 et appliquée sur des temps d'action compris entre 18 et 24 heures.
Le principe est le suivant : les bactéries sont inoculées par transfert sur le support à tester, puis le nombre de colonie de bactéries est mesuré une première fois à t=0h, et une deuxième fois à t=24h.
Pour évaluer le nombre de colonies bactériennes restantes sur les supports à tester à un temps donné, on les compte à l'aide d'une méthode de comptage de plaque.
On peut en déduire la valeur de la croissance des essais (notée G), définie comme suit :

$$F(log_{10}) = moy\ log\ T_{t24} - moy\ log\ T_0$$

formule dans laquelle $T_{t24}$ correspond au nombre de colonies bactériennes à 24h et $T_0$ correspond au nombre de colonies bactériennes juste après leur mise en contact avec le support testé.

La valeur de la croissance de souches comparatives témoins (notée F) est également déterminée et définie comme suit :

$$F(\log_{10})=\text{moy} \log C_{t24} - \text{moy} \log C_0$$

formule dans laquelle $C_{t24}$ correspond au nombre de colonies bactériennes à 24h et $C_0$ correspond au nombre de colonies bactériennes juste après leur mise en contact avec le support témoin.

On peut ainsi en déduire la valeur de l'activité bactérienne (notée A), définie comme suit :

$$A(\log_{10})=F - G$$

Les conditions expérimentales sont les suivantes :

- Le diluant utilisé est le peptone/sel (référence DIFCO, 218971) et la souche bactérienne employée est soit *Staphylococcus aureus* CIP 4.83, soit *Klebsiella pneumoniae* 368 CIP.
- La concentration de l'inoculum pour *Staphylococcus aureus* est de $3,8.10^5$ UFC/ml. La concentration de l'inoculum pour *Klebsiella pneumoniae* est de $1,23.10^6$ UFC/ml.
- Le support témoin est un textile 100 % coton non traité.

Les résultats sont reportés ci-après.

*Staphylococcus aureus*

| Temps d'incubation | 0h | | | | 24h | | |
|---|---|---|---|---|---|---|---|
| Réf échantillon | éprouvette | $C_0$ (UFC) | log ($C_0$) | moy log ($C_0$) | $C_{r24}$ (UFC) | log ($C_{t24}$) | moy log ($C_{t24}$) |
| **Témoin : textile 100% coton** | 1 | 36 000 | 4,56 | **4,45** | 23 500 000 | 7,37 | **7,42** |
| | 2 | 21 600 | 4,33 | | 29 800 000 | 7,47 | |

| Temps d'incubation | 0 h | | | | 24 h | | |
|---|---|---|---|---|---|---|---|
| Réf. échantillon | éprouvette | $T_0$ (UFC) | log ($T_0$) | moy log ($T_0$) | $T_{t24}$ (UFC) | log ($T_{t24}$) | moy log ($T_{t24}$) |
| **Exemple 6** | 1 | 52 000 | 4,72 | **4,62** | 1 270 | 3,10 | **2,64** |
| | 2 | 33 000 | 4,52 | | 150 | 2,18 | |

On en déduit l'activité antibactérienne suivante :

$$A(\log_{10})= 4,95.$$

Ces essais démontrent par conséquent que les supports obtenus conformément à l'invention peuvent également présenter, outre une activité antivirale significative, une activité bactéricide significative.

*Klebsiella pneumonie*

| Temps d'incubation | | 0h | | | 24h | | |
|---|---|---|---|---|---|---|---|
| Réf échantillon | éprouvette | $C_0$ (UFC) | log ($C_0$) | moy log ($C_0$) | $C_{t24}$ (UFC) | log ($C_{t24}$) | moy log ($C_{t24}$) |
| Témoin : textile 100% coton | 1 | 45 000 | 4,65 | 4,55 | 16 100 000 | 7,21 | 7,10 |
| | 2 | 27 900 | 4,45 | | 10 000 000 | 7,00 | |

| Temps d'incubation | | 0h | | | 24 h | | |
|---|---|---|---|---|---|---|---|
| Réf. échantillon | éprouvette | $T_0$ (UFC) | log ($T_0$) | moy log ($T_0$) | $T_{t24}$ (**UFC**) | log ($T_{t24}$) | moy log ($T_{t24}$) |
| Exemple 6 | 1 | 57 000 | 4,76 | 4,82 | 0 | - | - |
| | 2 | 75 000 | 4,88 | | 0 | - | |

On peut noter que les deux éprouvettes présentent une activité bactéricide.

Ces essais démontrent par conséquent, que les supports obtenus conformément à l'invention peuvent également présenter, outre une activité antivirale significative, une activité bactéricide significative.

Les exemples présents ci-dessus ne sont évidemment pas exhaustifs et d'autres supports de base pourront être envisagés sans sortir du champ de protection du brevet.

En particulier, le support de base pourra être un papier de sécurité haute durabilité objet de la demande de brevet FR 2 814 476, un papier impression/écriture, un calque ou un billet plastique.

**Revendications**

**1.** Support d'information destiné à être manipulé relativement fréquemment, **caractérisé en ce qu'**il contient :

- de 0,1 à 2 % en poids sec, par rapport à son poids total, d'au moins un virucide d'origine naturelle, ledit virucide étant la monolaurine, et
- au moins un agent humectant, ledit agent humectant étant du glycérol,

ledit support d'information étant **caractérisé en ce qu'**il s'agit d'un passeport, d'une carte d'identité, d'un permis de conduire, d'une carte d'accès, d'une carte de fidélité, d'une carte de photocopie, d'une carte de cantine, d'une carte à jouer, d'une carte à collectionner, d'un moyen de paiement, notamment d'une carte de paiement, d'un billet de banque, d'un bon d'achat ou d'un reçu, d'un ticket d'accès à des manifestations culturelles ou sportives, d'un certificat d'authenticité, ou encore d'un emballage, d'un livre, d'une carte géographique, d'une étiquette, d'une enveloppe ou d'un magasine.

**2.** Support d'information selon la revendication 1, **caractérisé en ce qu'**il contient de 0,5 à 1,5 % en poids sec dudit virucide d'origine naturelle par rapport à son poids total.

**3.** Support d'information selon l'une quelconque des revendications précédentes, **caractérisé en ce que** le rapport pondéral masse d'agent humectant sur masse de virucide est au moins égal à 1.

**4.** Support d'information selon l'une quelconque des revendications précédentes, **caractérisé en ce qu'**il contient en outre au moins un biocide bactériostatique et/ou bactéricide et/ou au moins un biocide fongistatique et/ou fongicide.

**5.** Support d'information selon l'une quelconque des revendications 1 à 4, **caractérisé en ce qu'**il est à base de matériaux cellulosiques, et en particulier de papier, ou à base de matériaux plastiques, et notamment de fibres synthétiques ou d'une feuille plastique.

**6.** Support d'information selon la revendication précédente, ledit support étant une feuille comportant un support multicouche coextrudé, laminé ou contrecollé, réalisé à partir d'au moins un matériau polymère.

7. Support d'information selon l'une quelconque des revendications précédentes, **caractérisé en ce que** ses propriétés virucides sont conférées *via* une encre, imprimée sur ledit support, ladite encre comprenant au moins ledit virucide d'origine naturelle.

8. Support d'information selon l'une quelconque des revendications précédentes, **caractérisé en ce que** ses propriétés virucides sont conférées *via* un vernis, notamment de surimpression, déposé sur ledit support, ledit vernis comprenant au moins ledit virucide d'origine naturelle.

9. Support d'information selon l'une quelconque des revendications précédentes, **caractérisé en ce qu'**il s'agit d'un billet de banque.

10. Procédé de fabrication d'un support d'information selon l'une quelconque des revendications précédentes, comprenant au moins l'étape consistant à mettre en contact, en présence d'au moins un agent humectant, un support de base avec ledit virucide d'origine naturelle, ou notamment un précurseur de celui-ci, dans des conditions propices à l'incorporation de celui-ci au niveau dudit support.

11. Procédé selon la revendication 10, **caractérisé en ce que** ladite mise en contact se fait par surfaçage dudit support de base avec une préparation contenant ledit virucide et un agent de surfaçage aqueux, ou par dépôt sur ledit support de base, d'un vernis de surimpression contenant ledit virucide.

12. Procédé de fabrication d'un support d'information, **caractérisé en ce qu'**au moins un virucide d'origine naturelle est synthétisé *in situ* au niveau d'un support de base formé par exemple de matériaux cellulosiques et/ou plastiques.

13. Procédé selon la revendication 12, **caractérisé en ce que** ledit virucide est la monolaurine synthétisée *in situ* par réaction d'acide laurique et de glycérol en présence d'un catalyseur, ledit catalyseur étant de préférence un catalyseur de type zéolite ou une lipase.

14. Procédé selon la revendication 13, comprenant au moins :

   a) le dépôt en surface dudit support de base, en présence d'un catalyseur, d'une composition contenant au moins de l'acide laurique et du glycérol, et
   b) le traitement consécutif de ce support à une température propice à la synthèse de monolaurine, le catalyseur étant de préférence présent dans le support de base mis en oeuvre en étape a) ou dans la composition contenant l'acide laurique et le glycérol.

15. Procédé selon la revendication 14, **caractérisé en ce que** ledit support de base est un support à base de matériaux cellulosiques, notamment du papier, et **en ce que** les étapes a) et b) sont réalisées simultanément aux étapes requises pour le couchage, l'enduction ou le surfaçage dudit support de base.

16. Procédé selon la revendication 14, **caractérisé en ce que** l'étape b) est réalisée simultanément à l'étape de séchage du papier couché, enduit ou surfacé.

17. Procédé selon la revendication 13, **caractérisé en ce que** ledit catalyseur est introduit à raison d'au moins 2 % en poids, par exemple au moins 5 % en poids, par rapport au poids total du mélange d'acide laurique et de glycérol.


**Patentansprüche**

1. Informationsträger, ausgelegt zur relativ häufigen Manipulation, **dadurch gekennzeichnet, dass** er enthält:

   0,1 bis 2 % im Trockengewicht bezüglich seines Gesamtgewichts von mindestens einem Viruzid natürlichen Ursprungs, wobei das Viruzid Monolaurin ist, und
   mindestens ein Feuchthaltemittel, wobei das Feuchthaltemittel aus Glyzerin ist,
   wobei der Informationsträger **dadurch gekennzeichnet ist, dass** es sich um einen Pass, einen Identitätsausweis, einen Führerschein, eine Zutrittskarte, eine Treuekarte, eine Fotokopierkarte, eine Kantinenkarte, eine Spielkarte, eine Sammelkarte, ein Zahlungsmittel, insbesondere eine Zahlungskarte, eine Banknote, einen Gutschein oder einen Empfangsschein, eine Eintrittskarte zu kulturellen oder sportlichen Veranstaltungen, ein Authentifizierungszertifikat oder auch eine Verpackung, ein Buch, eine geographische Karte, eine Etikette, einen Umschlag oder ein Magazin handelt.

2. Informationsträger nach Anspruch 1, **gekennzeichnet dadurch, dass** er 0,5 bis 1,5 % Trockengewicht des Viruzids natürlichen Ursprungs bezüglich seines Gesamtgewichts enthält.

3. Informationsträger nach einem der vorhergehenden Ansprüche, **gekennzeichnet dadurch, dass** das Gewichtsverhältnis der Masse des Feuchthaltemittels zur Masse des Viruzids mindestens gleich 1 ist.

4. Informationsträger nach einem der vorhergehenden Ansprüche, **gekennzeichnet dadurch, dass** er unter anderem mindestens ein bakteriostatisches Biozid und/oder ein Bakterizid und/oder mindestens ein fungistatisches Biozid und/oder Fungizid enthält.

5. Informationsträger nach einem der Ansprüche 1 bis 4, **gekennzeichnet dadurch, dass** er auf Zellulosematerialien und insbesondere auf Papier oder auf Kunststoffmaterialien und insbesondere auf synthetischen Fasern oder Kunststofffolie basiert.

6. Informationsträger nach dem vorhergehenden Anspruch, wobei der Träger eine Folie ist, die eine koextrudierte, laminierte oder verleimte mehrschichtige Basis aufweist, die von mindestens einem Polymermaterial ausgehend realisiert ist.

7. Informationsträger nach einem der vorhergehenden Ansprüche, **gekennzeichnet dadurch, dass** seine Eigenschaften als Viruzid von einer Tinte kommen, die auf seine Basis aufgedruckt ist, wobei die Tinte mindestens das Viruzid natürlichen Ursprungs umfasst.

8. Informationsträger nach einem der vorhergehenden Ansprüche, **gekennzeichnet dadurch, dass** seine Eigenschaften als Viruzid von einem Lack, insbesondere durch Überdruck, kommen, der auf die Basis aufgebracht ist, wobei der Lack mindestens das Viruzid natürlichen Ursprungs umfasst.

9. Informationsträger nach einem der vorhergehenden Ansprüche, **gekennzeichnet dadurch, dass** es sich um eine Banknote handelt.

10. Verfahren zur Herstellung eines Informationsträgers nach einem der vorhergehenden Ansprüche, umfassend mindestens den Schritt, bei dem eine Trägerbasis, bei Vorhandensein des mindestens einen Feuchthaltemittels, mit dem Viruzid natürlichen Ursprungs oder insbesondere einem Vorgänger desselben unter Bedingungen in Kontakt gebracht wird, die für die Aufnahme desselben auf Höhe der Basis günstig sind.

11. Verfahren nach Anspruch 10, **gekennzeichnet dadurch, dass** das In-Kontakt-Bringen durch Oberflächenbehandlung der Trägerbasis mit einem vorbereiteten Mittel, das das Viruzid und ein Mittel zur wässrigen Oberflächenbehandlung enthält, oder durch Aufbringen eines Überdrucklacks, der das Viruzid enthält, auf der Trägerbasis stattfindet.

12. Verfahren zur Herstellung eines Informationsträgers **gekennzeichnet dadurch, dass** mindestens ein Viruzid natürlichen Ursprungs *in situ* auf Höhe einer Trägerbasis, die beispielsweise aus Zellulose- und/oder Kunststoffmaterialien gebildet ist, synthetisiert wird.

13. Verfahren nach Anspruch 12, **gekennzeichnet dadurch, dass** das Viruzid *in situ* durch Reaktion von Laurinsäure und Glyzerin bei Vorhandensein eines Katalysators synthetisiertes Monolaurin ist, wobei der Katalysator vorzugsweise ein Katalysator vom Zeolithtyp oder eine Lipase ist.

14. Verfahren nach Anspruch 13, umfassend zumindest:

a) das Aufbringen einer Zusammensetzung, die zumindest Laurinsäure und Glyzerin enthält, bei Vorhandensein eines Katalysators auf die Oberfläche der Trägerbasis und
b) die darauffolgende Behandlung der Basis bei einer für die Synthese des Monolaurins geeigneten Temperatur, wobei der Katalysator vorzugsweise in der in Schritt a) angefertigten Trägerbasis oder in der Zusammensetzung, die die Laurinsäure und das Glyzerin enthält, vorhanden ist.

**15.** Verfahren nach Anspruch 14, **gekennzeichnet dadurch, dass** die Trägerbasis eine auf Zellulosematerialien, insbesondere auf Papier, beruhende Basis ist, und dass die Schritte a) und b) gleichzeitig mit Schritten, die zur Aufeinanderschichtung, Beschichtung oder Oberflächenbehandlung der Trägerbasis erforderlich sind, ausgeführt werden.

**16.** Verfahren nach Anspruch 14, **gekennzeichnet dadurch, dass** Schritt b) gleichzeitig mit einem Schritt des Trocknens von aufeinandergeschichtetem, beschichtetem oder oberflächenbehandeltem Papier ausgeführt wird.

**17.** Verfahren nach Anspruch 13, **gekennzeichnet dadurch, dass** der Katalysator mit mindestens 2 Gewichts-%, beispielsweise mindestens 5 Gewichts-% bezüglich des Gesamtgewichts der Mischung der Laurinsäure und des Glyzerins eingeführt wird.

## Claims

**1.** An information medium intended to be handled relatively frequently, **characterized in that** it contains:

- from 0.1 to 2% by dry weight, relative to its total weight of at least one virucide of natural origin, said virucide being monolaurin, and
- at least one humectant, said humectant being glycerol,

said information medium being **characterized in that** it is a passport, an identity card, a driver's license, an access card, a loyalty card, a photocopier card, a canteen card, a playing card, a collectable card, a means of payment, in particular a payment card, a banknote, a purchase slip or a receipt, a ticket for entry into a cultural or sporting event, a certificate of authenticity, or else packaging, a book, a geographic map, a label, an envelope or a magazine.

**2.** The information medium as claimed in claim 1, **characterized in that** it contains from 0.5 to 1.5% by dry weight, of said virucide of natural origin, relative to its total weight.

**3.** The information medium as claimed in any one of the preceding claims, **characterized in that** the mass of humectant to mass of virucide weight ratio is at least equal to 1.

**4.** The information medium as claimed in any one of the preceding claims, **characterized in that** it also contains at least one bacteriostatic and/or bactericidal biocide and/or one fungistatic and/or fungicidal biocide.

**5.** The information medium as claimed in any one of claims 1 to 4, **characterized in that** it is based on cellulosic materials, and in particular paper, or on plastic materials, and in particular synthetic fibers or a plastic sheet.

**6.** The information medium as claimed in the preceding claim, said medium being a sheet comprising a co-extruded, laminated or glued multilayer medium, made from at least one polymer material.

**7.** The information medium as claimed in any one of the preceding claims, **characterized in that** its virucidal properties are conferred via an ink, printed onto said medium, said ink comprising at least said virucide of natural origin.

**8.** The information medium as claimed in any one of the preceding claims, **characterized in that** its virucidal properties are conferred via a varnish, in particular an overprint varnish, deposited on said medium, said varnish comprising at least said virucide of natural origin.

**9.** The information medium as claimed in any one of the preceding claims, **characterized in that** it is a banknote.

**10.** A method for producing an information medium as claimed in any one of the preceding claims, comprising at least the step consisting in bringing a basic medium into contact, in the presence of at least one humectant, with said virucide of natural origin, or in particular a precursor thereof, under conditions suitable for the incorporation thereof at the level of said medium.

**11.** The method as claimed in claim 10, **characterized in that** said bringing into contact is carried out by surface-treating said basic medium with a preparation containing said virucide and an aqueous surface-treating agent, or by depositing onto said basic medium an overprint varnish containing said virucide.

**12.** A method for producing an information medium, **characterized in that** at least one virucide of natural origin is synthesized in *situ* at the level of a basic medium formed, for example, from cellulosic and/or plastic materials.

**13.** The method as claimed in claim 12, **characterized in that** said virucide is monolaurin synthesized *in situ* by reacting lauric acid and glycerol in the presence of a catalyst, said catalyst being a catalyst of zeolite type or a lipase.

**14.** The method as claimed in claim 13, comprising at least:

a) depositing at the surface of said basic medium, in the presence of a catalyst, a composition containing at least lauric acid and glycerol, and
b) subsequently treating this medium at a temperature suitable for the synthesis of monolaurin, said catalyst being preferably present in the basic medium used in step a) or in the composition containing the lauric acid and the glycerol.

**15.** The method as claimed in claim 14, **characterized in that** said basic medium is a medium based on cellulosic materials, in particular paper, and **in that** steps a) and b) are carried out simultaneously with the steps required for the layering, coating or surface-treating of said basic medium.

**16.** The method as claimed in claim 14, **characterized in that** step b) is carried out simultaneously with the step of drying the layered, coated or surface-treated paper.

**17.** The method as claimed in claim 13, **characterized in that** said catalyst is introduced in a proportion of at least 2% by weight, for example at least 5% by weight, relative to the total weight of the mixture of lauric acid and glycerol.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03084326 A
- US 20080279959 A
- EP 0059056 A
- FR 2814476

**Littérature non-brevet citée dans la description**

- **PEREIRA C.C.B. ; DA SILVA M.A.P. ; LANGONE M.A.P.** Enzymatic synthesis of monolaurin. *Applied biochemistry and Biotechnology,* 2004, vol. 113 (116), 433-445